# EUROPEAN PATENT APPLICATION

(11) **EP 2 759 262 A1**
(43) Date of publication of application: **30.07.2014**
(21) Application number: 12795062.4
(22) Date of filing: 24.09.2012
(51) Int. Cl.: A61B 5/11

(54) **SYSTEM AND METHOD FOR MONITORING AND REGISTERING THE INCLINATION AND DIRECTION OF AN INDIVIDUAL**

(30) Priority: 23.09.2011 PT 2011105901
(71) Applicant: Tomorrow Options - Microelectronics, S.A., 4050-448 Porto (PT)
(72) Inventor: REIS CUNHA, Sérgio, P-4250-340 Porto (PT); MACHADO MAGALHÃES COSTA E SILVA, Pedro Manuel, P-4350-171 Porto (PT); DO CUBO NEIVA, João Henrique, P-4200-472 Porto (PT); ALMEIDA MOREIRA PINTO, Jorge Miguel, P-4435-773 Baguim do Monte (PT); CHRISTIAN MARIE OOMEN, Willem Reinoud, Spurstowe Terrace, E8 1FD London (GB); FLORES FERREIRA DOS SANTOS, Paulo António, 4450-251 Matosinhos (PT)
(74) Representative: Silvestre de Almeida Ferreira, Luís Humberto
(86) International application number: PCT/IB2012/055075
(87) International publication number: WO 2013/042097

(57) **Abstract**

The system comprises a monitoring device (1), one acquisition unit (3) and respective method for monitoring and registering the position of an individual (2).

This disclosure is intended for the following applications: 1) prevention or assistance in the treatment of pressure ulcers (or decubitus ulcers), common in immobile patients (2) or wheelchairs users, 3) monitoring of patients suffering from dementia (which makes them do some actions (for instance: getting up from a chair) without being aware of them, and that can suffer serious accidents due to that), 4) fall detection and 5) involuntary body movements (e.g. epileptic attack).

The monitoring device (1) comprises an inertial system, with 3 or more accelerometers; a directional system, with 3 or more magnetometers; a communication module; and a processor module to obtain and communicate the inclination and direction of the device, when coupled to an individual.

## Description

### Technical field

The disclosure concerns a system and method for monitoring and registering the position (relative to a plane) of an individual.

Its use is strictly related to the following applications: prevention, mainly through an alarm to reposition the patient's body within a set period of time for each case, of pressure ulcers (or decubitus ulcers), common in patients who stay immobile for longs periods of time and deprived of movements, people limited to wheelchairs, monitoring of patients that suffer from dementia, detection of falls and involuntary body movements (e.g. epileptic attack), in order to assure their physical welfare and/or a timely intervention from the care takers, preventing bigger damages.

The disclosure comprises medical and electronical domains.

### Summary

The disclosure describes a monitoring device for patients with movement related difficulties and is characterized for performing the monitoring and temporal registering of the position of an individual (2, 5) relative to a plane.

An embodiment of the disclosure comprises an inertial system (1, 4), particularly three or more accelerometers, combined with magnetometers.

In a further embodiment the disclosure comprises three or more magnetometers.

In a further embodiment the disclosure comprises additionally an energy module, a communication module with or without wires for one acquisition unit (3, 6), and an audio warning module.

In a further embodiment the disclosure, the energy module comprises a battery, a battery charger and a sub-module for energy regulation and monitoring.

In a further embodiment the disclosure describes still a method for monitoring patients with movement related difficulties, characterized for performing the monitoring and temporal registering of the position of an individual (2, 5) relative to a plane through an inertial system (1, 4), particularly three or more accelerometers combined with magnetometers, particularly three or more magnetometers.

In a further embodiment the device is capable of monitoring bedridden patients or in wheelchairs and respective prevention of pressure ulcers through sending an alarm regarding the need of changing the body position (repositioning) in the set period for each case, being placed on the individual's torso (2) or on the thigh or on the waist (14) of the individual (5), or patients who suffer from dementia or motor limitation - as patients in wheelchairs, for instance - and respective prevention of accidents and fall or involuntary body movements.

In a further embodiment, the device is capable of gathering and storing all the relevant information of the temporal evolution of the position of the individual for posterior analysis.

In a further embodiment, the device is capable of, by determining the patient's position, determining indirectly the area of the body that is under pressure and the affected, critical areas of the body, assuming the inclination of the back of the bed is changed whenever the mentioned body inclination, especially from the torso, particularly forward, is maintained for more than the predetermined time period. In an equivalent way, keeping the body's lateral inclination, especially from the torso, for a predetermined time period, allows to infer the inclination of the back of the bed. In another way, keeping the body's inclination, laterally or forward, especially from the torso, for a predetermined time period, allows to infer the inclination of the back of the bed.

In a further embodiment, the device is capable of, after detecting a change in the position of the individual, or after a maximum predefined time in the same position, sending a local alarm, a remote alarm or both.

In a further embodiment, the device is capable of alerting that the individual is being placed in a position previously defined as inadequate.

In a further embodiment, the device is capable of alerting when the individual is placed in a determined position and returns immediately to the previous position, in a short period of time and considered insecure, motivating a new intervention to reposition the patient to a safe position. This execution is particularly important because sometimes, even when registered all the repositioning activity, ulcers occur and are believed to come from the returning to previous positions within an insufficient period for the recovering of the tissues. This situation leads to medical problems of difficult resolution, without the real information as the one we here define.

In a further embodiment, in monitoring bedridden patients (2) or in wheelchairs and respective pressure ulcers prevention, after a maximum predefined time in the same position, or while monitoring patients suffering from dementia (5), after the detection of change of the individual's position, or in fall prevention, when is detected that the patient fell, or when the device detects involuntary body movements, a local alarm, a remote alarm or both are sent.

The disclosure proposes a low cost and cost-effective solution, allows coverage to a high percentage of patients, if not all, has despicable costs, minute size and the maintenance is minimal.

### Background

Pressure ulcers (PU), also called decubitus ulcers or bedsores, are lesions caused by the compression of a certain part of the body, usually those containing bony prominences. These are defined as necrotic tissue located areas which tend to develop when a tissue is compressed between a bony prominence and an external surface for a prolonged time.

Common in bedridden patients for a long period, or even short periods in patients with risk factors (e.g.: diabetes, overweight, elderly) and movement deprived, causing a clinical picture of compression, ischemic injury and consequent tissue destruction.

Apart from the pressure related to duration, intensity and tissular tolerance, other risk factors contribute directly or indirectly to the development of the PU, and these risks are higher in Intensive Care Units, due to the characteristics of the patients in this sector.

There are several methods proposed whose purpose is to prevent or treat pressure ulcers. These methods are based on reduction equipments or direct monitoring of the pressure, as for instance air mattresses, articulated bed or systems for monitoring pressure, using pressure sensors.

The existing solutions based on air mattresses or beds with functionalities for reducing the pressure in certain areas of the body have sometimes high costs and, on the other hand, it is necessary to sum the high maintenance and reduced flexibility of these systems (e.g.: storage), which makes such solutions not practical if it is necessary to cover a high percentage of bedridden population of an health institution.

The indirect costs of pressure ulcers treatment are significant. However, there are other related costs which are relevant: occupying beds in hospitals, staffing, loss of working days, etc.

The existing equipments geared for prevention and treatment of pressure ulcers are based in measuring or redistributing the pressure in certain areas of the body. These equipments are inherently linked to the current technology for measuring pressure that, although in fast growing, is still based on sensors that have a limited life cycle and high costs. These solutions have also several limitations regarding feasibility, portability and maintenance.

The proposed disclosure distinguishes itself from this type of devices, since it does not measure the pressure intensity but instead it uses a combination of measurement of the body position (using an inertial system) with its duration, in order to prevent a long exposure to pressure of each critical area of the body.

Another aspect to point out is the fact that the proposed disclosure is complementary to some of the pressure reduction technologies, even contributing to its better use, due to allowing an historical analysis of the patients' activity.

The proposed device allows to detect the state changes critical for these individuals (sitting, up or walking) with an extremely reduced cost, as well as its extremely discrete use, which makes possible a comfortable monitoring for the patient.

The system is groundbreaking because, according to the knowledge of the authors, there is not any system in the market with this purpose and this approach (almost all are focused in measuring or reducing the pressure).

The disclosure proposes a low cost and cost-effective solution for a worldwide known problematic (e.g. in 2011 the British public health system spent 4% of its annual budget on the treatment of pressure ulcers).

Due to its low cost of application and maintenance, the disclosure allows to cover a high percentage of patients, if not all, that are in a potential risk situation in an health institution, because its price can allow, if necessary, to have one device in each bed, i.e., monitoring all the patients and probably spend less than the current situations that serve only few. In line with this, it allows increasing the auxiliary staff or nursing staff efficiency no matter what the patients/staff ratio is, maybe in some cases reducing the patients/staff ratio, and contributing to a better management without compromising the patients' health.

It is widely accepted and supported by many studies on the matter that a diligent follow-up of the state of an individual in reduced mobility situations results in the prevention of most pressure ulcers. However, there are situations in which this type of follow-up is not feasible, for instance, institutions with a high ratio patients/staff. The proposed disclosure's purpose is to help health professionals, making an automatic and transparent monitoring of the patients and sending programmable alerts according to the particular state of each patient.

The problem that this device intends to solve is to decrease significantly the human error, common in hospitals due to their intrinsic characteristics: many staff shifts (as they work 24 hours), regular stress situations which makes them redefine immediate priorities and forget basic procedures, bad circulation and management of information, lack of centralization of resources, etc. As a matter of fact, being this one of the 5 main clinical errors, it is why the approach to this problem should be done in a completely different way from the current ways, for they only act in the specific local of the patient, while the way proposed here involves all the extensive environment, contributing effectively to the immediate action of the most available nurse and not only the nurse responsible for the patient.

A complementary problem that the device also intends to solve is allowing the staff of healthcare units to occupy their time only with the patients that did not changed position within the defined safe time, thus increasing their efficiency, since that in the case of the patients that have changed their position by their own means and in case they are in a safe position no alarm will be sent for intervention. On the other side, in cases where patients have some ability to move but for any reason are not in full possession of their faculties (e.g.: under the effect of medicine), the local alarm, if active, will alert them to, by their own means, change position, saving the staff of doing this task.

### General description

The disclosure comprises of a device and method for monitoring and registering the position (relative to a plane) of an individual and the respective duration.

There are two basic elements which are critical for the functionality of the respective method. An accelerometers and magnetometers module to determine the relative position of the individual and one communication module, responsible for the communication to the external unit.

The developed method uses an inertial system, composed of three accelerometers and three magnetometers, which allows determining the position relative to a plane, through trigonometric relationships.

The duration of a position is also acquired, alerting to a prolonged exposure of a certain area of the body to a certain level of pressure. The obtained data is sent to a receiver unit, through the communication module, so that it can be visualized.

The position is acquired by the device (1) through an inertial system, placed on the individual's torso (2), and the obtained data is sent to an acquisition unit (3). The device also has a programmable alarm, for local alerting. In a preferential and accessory way to this execution, the position information can be complemented by the directional system.

The gathered and stored information is also relevant for the analysis of the bed occupation in hospitals.

The device use can be extended beyond hospitals, as due to its dimensions, ease of use and versatility, it also has application in domestic environment, nursing homes, assistance clinics, senior clinics, or any other structure or organization that includes people at risk of developing pressure ulcers.

Most scientific literature and medical directives consider that the most effective prevention process to avoid pressure ulcers is to change the patient's position regularly, avoiding that the same area of the body stays in contact with the bed or seat for too long (note: this maximum time is defined by the doctor or nurse for each patient in particular). It is also commonly considered in the mentioned literature that any bedridden patient should, at least, change position every two hours.

There are several intrinsic and extrinsic factors that contribute to the development of pressure ulcers and that influence the patient's risk degree to develop a pressure ulcer.

The intrinsic factors include, for instance, the general state, the nutritional state or the age of the individual.

The exposure of a certain area of the body to pressure during a certain time interval is an extrinsic factor; this is one of the targets of the proposed disclosure. That is, according to the patient's risk degree, he should not have the same areas of the body under pressure for more than a determined time limit (defined by the doctor for each particular case). For that reason, when close to the time limit, the patient must change position (if he is capable) or he must be helped to do it.

This last aspect is determinant for the inclusion of pressure ulcers in the list of clinical errors, since that, particularly in hospitals, nurses or auxiliaries forget to change the patients' position. Apart from that, as there are several shifts changes in healthcare establishments, not always the information is passed in the most convenient way, provoking procedure omissions.

In a groundbreaking way, the device allows decreasing the propensity of a certain individual developing a pressure ulcer in a certain area of the body, by monitoring the time interval and the individual's position.

In an embodiment for pressure ulcers, the device determines the patient's position using the accelerometers module (determining indirectly the area of the body that is under pressure, for instance, through simple heuristic or biomedical models more or less sophisticated) and count the time in which the patient is not moving. When this time is close to the maximum time defined by the doctor or nurse an alarm is sent, locally (can be turned off) and remotely, to someone that will change the patient's position. Preferably, the device will be placed in the upper anterior part of the thorax, by the neck basis.

In the case of bedridden patients, the position is measured relative to the plane of the bed the patient is in. Since the back of the beds can be adjustable, the device adjusts automatically the critical areas which are susceptible of developing ulcers with the change on the inclination of the back of the bed. This is done through a programmable maximum time interval in which the individual is inclined with a certain angle. If this remains with a certain inclination in a bigger time than the one programmed, the device assumes that the inclination of the back of the bed was changed to the current inclination and recalculates the critical areas of the body now affected.

Critical areas of the body which are under pressure are determined for each orientation. The sequence of filtered measurements allows calculating the accumulated recent time in which each area is under pressure. Once an area exceeds the maximum time under pressure (configurable time), alarms are generated as local acoustic warnings and/or by sending an alarm signal to a central computer (using the local wireless communication system).

With the increase of the elderly population, the number of dementia tends to increase. Among several symptoms, some stand out like disorientation, propensity to wander and a high attention deficit, mainly on elderly individuals, potentiating situations that can compromise their physical integrity. This kind of patients needs constant watch, which is not always feasible.

There are many methods with the purpose of preventing this behaviour, including physical barriers, sedatives, constant watch or absolute position (in space) monitoring systems. However, many of these methods or devices have high costs

(electronic monitoring systems, individual monitoring by an health auxiliary) or cause the degradation of the physical condition of the individual (use of sedatives).

The proposed device allows detecting the changes of position and of state of a certain patient, as for instance, the transition from sitting down to standing up and walking.

In an embodiment for dementia patients, the position is acquired by the device through an inertial system (4). The device can be placed on the thigh or thorax, so that it is possible to detect the transition of the state of dementia patients.

In a further embodiment, in each registered change, there is a local and/or remote alarm, alerting the person responsible for the patient that he is in a possibly dangerous situation, enabling a continuous and remote monitoring of many patients in an hospital or nursing home.

Wheelchairs users suffering from vertebral column lesions and that have few or no sensibility in the lower part of the body, often forget to change position to relieve some areas of the body (e.g.: buttock) exposed to the body weight for too long. This situation shows the need to alert them locally that they should change the support area when the defined maximum time in the same position has been reached.

The developed device allows determining the position of an individual's body, as well as the duration of the position, alerting locally and or remotely when the defined maximum time for each position has been exceeded.

In an embodiment for wheelchair patients, when placed in the correct area of the patient's body (thorax by the neck basis (1) or waist (14), according to the kind of disability/limitation of the patient), the device allows distinguishing which areas of the buttocks are in contact with the wheelchair and are susceptible of being subject to exposure of a certain level of pressure.

Particularly, the detection of the user maintaining the same position can be done through the detection of the variation of the inclination and/or orientation of the device beyond intervals of predetermined dimension.

Wounds are common in bedridden patients. When they appear, it is necessary to treat them immediately to prevent infections. After the detection and for a better treatment, the patient must not place the injured area of the body under pressure. According to the presence of other pathologies it can be necessary to restrain certain positioning of the patient over the bed.

In an embodiment for patients with "forbidden" contact areas, the device allows alerting that the patient is being place in a position previously defined as inadequate for the patient's particular condition (e.g.: the doctor determines that a patient must not be placed under his left side, in case anyone puts him, inadvertently in that position, an alarm will sound).

Patient's falls, in hospitals or at home, are an important subject and to which the scientific community has been progressively paying more attention. Apart from creating lesions on patients, these falls are a primary factor in expenses increase. Currently, there are not sufficient nurses, or efficient nursing staff organization, so that all patients are watched, and so, the fall rate is very high in many countries of the world. Approximately half of the patients who suffer a fall have no ability to stand up on their own or the same ability is not detected in time.

In an embodiment for fall detection, with the permanent data acquisition from magnetometers and accelerometers, the device detects the existence of an impact on the body, above a previously defined level of acceleration and/or orientation signal. After the detection of that impact, a frequency analysis is made, during a previously defined time interval, to determine if the detected impact was due to a fall (being detected the existence of high energy in certain frequencies, simultaneously with the movement during the analysis) or just an abrupt movement from the user.

With the analysis of the impact frequencies' components and the movements performed by the body, most of the falls are detected.

From the many pathologies that can lead to involuntary body movements epilepsy stands out.

The epileptic state is a medical emergency because the person has seizures and intense muscular spasms, cannot breathe properly and has extensive electrical discharges (diffuse) on the brain. The fast and violent muscular spasms have a risk of wounds in case of body impacts and can even produce bone fractures. The sudden loss of conscience can cause severe lesions by falls and accidents.

In the disclosure, with the permanent analysis of the patient's orientation, it is possible to detect that the patient shows an unstable and random behaviour during some seconds, allowing to alert locally and remotely the request for help.

In an embodiment for the detection of involuntary body movements, the detection of a behaviour classified as unstable and random is made through a frequencies analysis under the three measuring axes of the accelerometer. If the energy, in a previously defined frequencies range, is higher than the predefined limit, an alarm is sent.

Using the communication network properties used by the monitoring device to send data to the acquisition unit, it is possible to determine the location in the space of the monitoring device, determining thus the spatial location of the patient.

In an embodiment for the spatial location of the user, to determine the spatial location of the monitoring device it is detected which is the access point that the same uses to communicate with the acquisition unit. If the access point to which it communicates is not equal to the previously configured access point, the conclusion is that the monitoring device has changed position and the patient has changed location (e.g.: has left the infirmary and, by disorientation, has entered a different infirmary).

The movement detection (e.g.: walking) of a bedridden patient is important to be made as soon as possible after he has left his bed, in order to minimize the risk of fall or accident to which the patient may be subjected to.

In an embodiment for the gait detection by the user, the disclosure enables the detection of movement of a patient, when he has the monitoring device applied in any part of the body. The detection is made through the analysis of the frequencies' components of the accelerometers. If the energy of a frequencies range, previously defined, exceeds a limit also previously defined, the conclusion is that there is a gait pattern present, meaning that the patient is moving.

### Brief Description of the Figures

The following figures provide preferred embodiments for illustrating the description and should not be seen as limiting the scope of the disclosure.

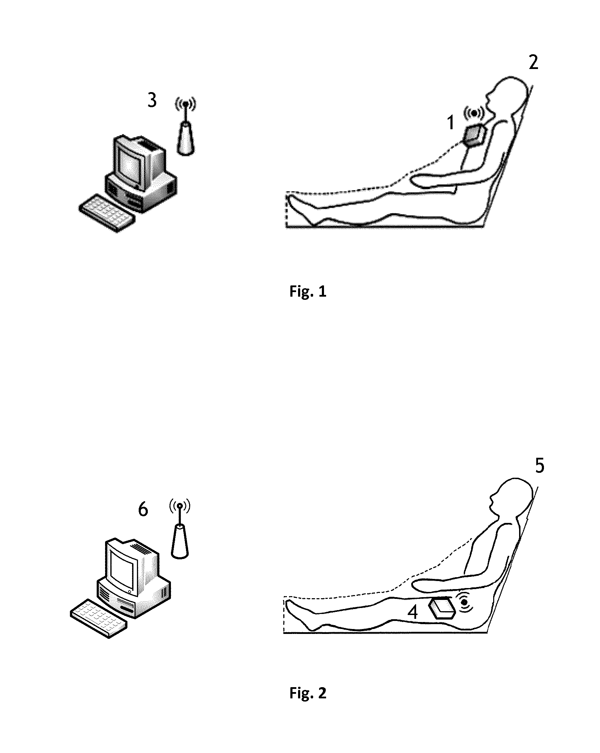

**Figure 1****:** Schematic representation of the patients monitoring device where
   (1) represents the remote unit or monitoring device, which includes the inertial system, located under the individual's torso,
   (2) represents the individual being monitored, and
   (3) represents the acquisition unit or device.
**Figure 2****:** Schematic representation of the patients monitoring device where
   (4) represents the remote unit or monitoring device, which includes the inertial system, located on the individual's thigh,
   (2) represents the individual being monitored, and
   (3) represents the acquisition unit.

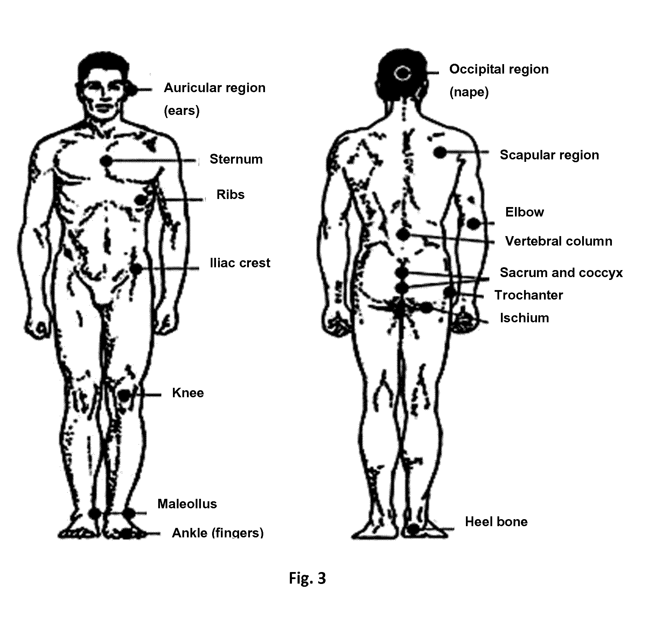

**Figure 3****:** Schematic representation of the areas of bigger incidence of Pressure Ulcers on the Ventral and Dorsal View of the human body.

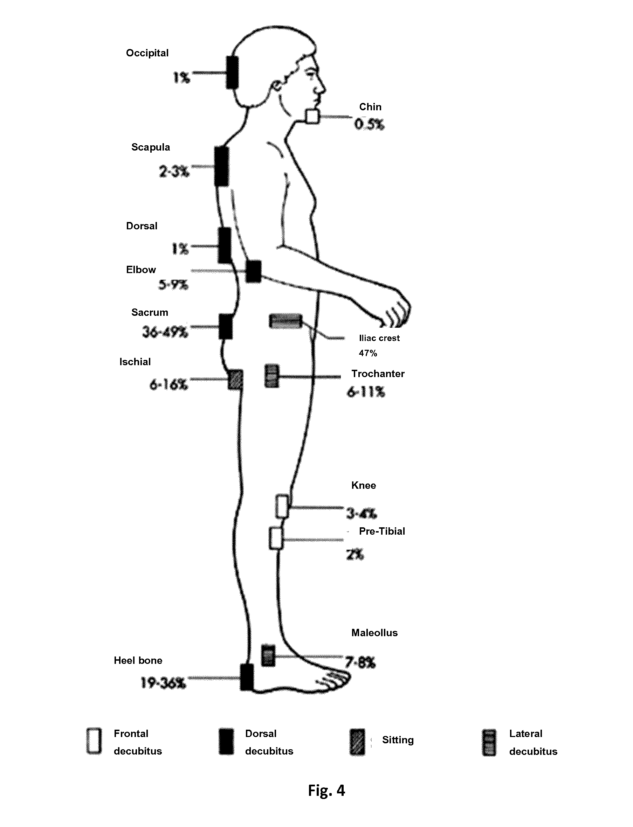

**Figure 4****:** Schematic representation of ulceration frequencies by area.

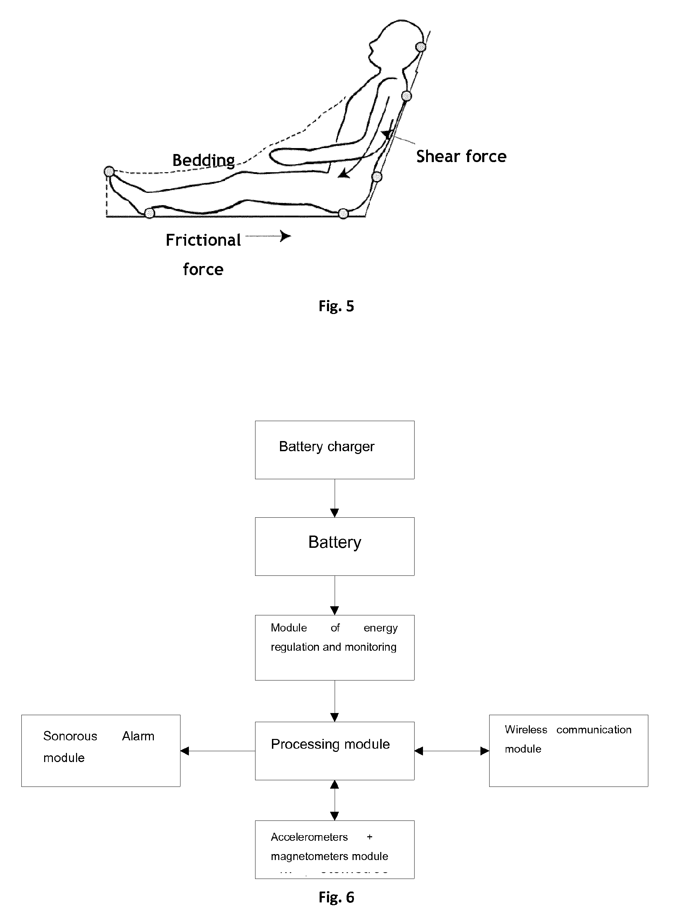

**Figure** 5: Schematic representation of the pressure points, Frictional force and Shear Force.
**Figure 6****:** Functional schematic of the remote unit which comprises the inertial system.

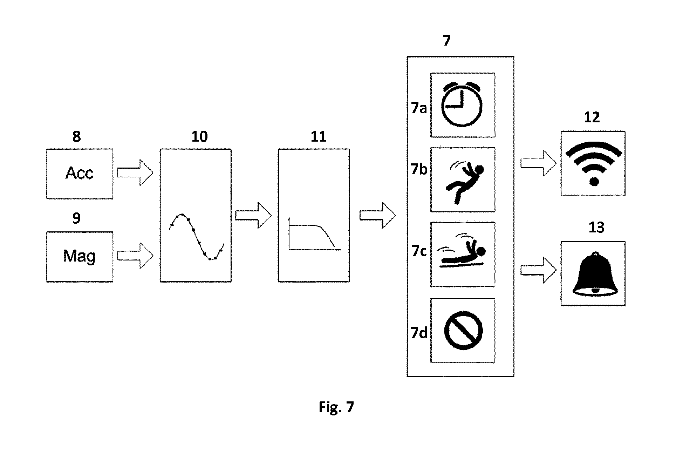

**Figure 7****:** Block diagram where
   (7) represents the data processing module, (7a) represents a temporization sub-module, (7b) represents a fall detection sub-module, (7c) represents a seizure sub-module, (7d) represents a "forbidden" positions detection sub-module,
   (8) represents a module with an inertial system with accelerometers,
   (9) represents a module with a directional systems with magnetometers,
   (10) represents a signal collection module, particularly of sampling with analogical/digital conversion,
   (11) represents a signal treatment module, particularly with loss-pass filter,
   (12) represents a remote alarm module, sent particularly through a mobile data network of the communication protocols Wifi, Bluetooth, Zigbee or RF, and
   (13) represents an alarm module local to the device, particularly sonorous and/or luminous.

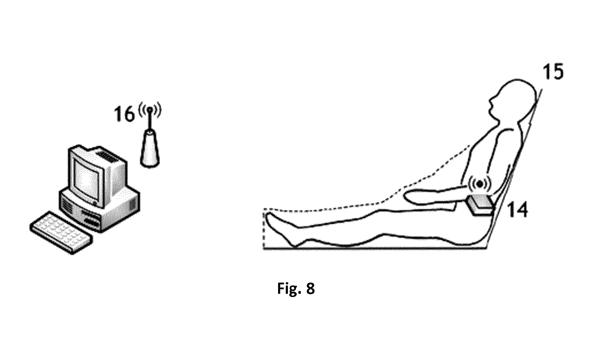

**Figure 8****:** Schematic representation of the device for patient monitoring where
   (14) represents the remote unit or the monitoring device, which includes the inertial system, located on the patient's waist,
   (15) represents the individual being monitored, and
   (16) represents the acquisition unit.

### Detailed description

The device uses the measurements from three accelerometers positioned in orthogonal directions among them and three magnetometers, also positioned in orthogonal directions among them, to determine the device's orientation (and the body part to which it is fixed) in space. The three accelerometers and the three magnetometers are of low cost and can be all included in the same integrated circuit.

In certain, less demanding, applications the number of accelerometers and/or magnetometers can be decreased, with the inconvenience of not obtaining the measurements of 3 axes. In certain, more demanding, applications the number of accelerometers and/or magnetometers can be increased, with the need of making the obtained measurements compatible (e.g.: by an heuristic or statistically).

The combination of at least three accelerometers and three magnetometers aligned allows the full characterization of the device's attitude. The position of the device can be obtained using only three or more accelerometers, but nothing can be inferred regarding its orientation. The use of three or more magnetometers allows the determination of the orientation of the device regarding Earth's magnetic north pole.

In the current description it is understandable that the term accelerometer or magnetometer concerns measuring devices comprising one direction. It is indifferent if they use 3 discrete devices in 3 directions or one discrete device of 3-axes, for instance.

To calculate this orientation, the device's microprocessor applies digital filters in cascade to the accelerometers and magnetometers to obtain measurements of the three orthogonal axes with a bandwidth inferior to 1 Hz. This allows eliminating significantly the noise level to which the raw measures are subjected, as well as eliminating possible fast vibrations to which the device may be subjected.

The joint analysis of the filtered measurements allows calculating the previously referred orientation, using trigonometric relationships. Although using low cost accelerometers and magnetometers, the orientation is determined with an error inferior to one degree.

The way the measurements of the accelerometers and magnetometers are filtered allows determining the orientation with enough precision to characterize several postures and map with rigour the critical areas under pressure.

Using the detection of the body's orientation, when the monitoring device is placed in a certain part of the body, as for instance in the thorax, the contact areas between the body and the bed can be inferred. With this disclosure, the time for each contact area is calculated. The contact areas are classified by area of bigger incidence of pressure ulcers, based on literature. Whenever an inferred contact area is no more in contact with the bed, the accumulated time value for that position will decrease gradually. If the body returns to a previously used position, i.e., if the accumulated time of an inferred area is not null yet or is not surpassing a predefined threshold yet, the system sends a local or remote alarm, considering the position as inadequate. In an embodiment the ratios of accumulation and disaccumulation of pressure are different, allowing to define that, for instance, it is only needed to relief a certain area of the body for 15 minutes, to recover from one hour of pressure. In an embodiment, the ratios are different according to the area of the body, allowing to define that, for instance, it is possible to recover one hour of pressure in just 10 minutes in the case of legs, but 20 minutes will be needed to recover from the pressure received on the back. In an embodiment, it is possible to have simultaneously different ratios of accumulation and disaccumulation and according to the area of the body.

For example, if the patient is in the decubitus lateral position for 10 minutes, after being in the decubitus dorsal position for two hours, and if the patient returns to the decubitus dorsal position again the system sends an alarm. In an embodiment is the detection of a larger amount of time than a predetermined time threshold in the current position, after which the device considers the position previous to the current one as "forgotten". The return to the position previous to the current one before the predetermined threshold expires, triggers a local and/or remote alarm.

In an embodiment the body is divided by areas, as many as necessary for an adequate characterization of the pressure. The inference of the pressure distribution for the several areas for a certain orientation of the body can simply be calculated through correlations previously fixed, for instance in the position lying down at 35%, of the pressure of the body is communicated to the patient's back. Alternatively, the inference of the pressure distribution for the several areas for a certain orientation of the body can simply be calculated through bioinformatic models, for instance, by the calculation of finite elements, characterizing the pressure in each cm² of the patient' skin.

In the case of PU, the device has electronic sensors and provides quantitative data about the patient's position and the time that the patient is on that same position. The time is adjustable to each patient and can be programmed according to the degree of development of the pressure ulcers. The device issues a sound alarm signal that serves as a warning and helps the nurses to know when the patient should change position. If the patient has mobility, physical and neurological ability, he can change position by himself when the alarm sounds. In case of patients with dementia, the device alerts when there is a change of state of the user, sending a local sound signal or a remote alert.

The above described embodiments are obviously combinable. It is a simple, small and easy to use device. The device is to be used by technicians, health professionals, nurses or other auxiliary staff and is placed directly on the patient. You can easily attach it on the skin in several parts of the body. Best places are superior part of the thorax or the waist for patients sitting or lying down; or the thigh (through a pocket) to determine when a patient gets up. The device uses a rechargeable battery, its power supports the device's functioning preferably for at least 10 to 15 days; or non-rechargeable preferably during at least 6 months. The data and the settings of the devices are communicated to and from a central computer to each installation. The communications are made under radio technology, as ZigBee, Wi-Fi, Bluetooth, Social Alarms RF and so on.

The gathered and processed information is sent to a data base to be analysed and assessed through Software.

Since this is a portable device, it can be used in health units, in the home of the patient or in any other place, therefore being much comfortable.

The disclosure is obviously in no way restricted to the exemplary embodiments described and the skilled person will contemplate modifications without departing from the scope of the disclosure as defined in the claims.

The following claims define additional embodiments of the disclosure.

## Claims

1. System to monitor and register the position of an individual (2, 5), comprising a monitoring device (1, 4) which comprises:
- an inertial system with 3 or more accelerometers,
- a communication module, and
- a processing module configured to obtain and communicate the inclination and direction of the monitoring device, when coupled to an individual, such that the inclination and direction of the individual is obtained and communicated.

2. System according to the previous claim, comprising a directional system with three or more magnetometers.

3. System according to any of the previous claims, comprising an energy module, a communication module, with or without wires, of communication to an acquisition unit (3, 6), and a sound alarm module.

4. System according to claim 1 wherein the energy module includes one battery, one battery charger and a sub-module for regulation and energy monitoring.

5. System according to any of the previous claims configured, by determining the individual's inclination and direction, to determine indirectly one or more of: the individual's position, the area of the individual's body under pressure, or the critical areas of the individual's body affected by pressure.

6. System according to any of the previous claims wherein the mentioned monitoring device (1, 4) is programmed, after detecting the individual's change of position, or after a predefined maximum time of the individual in the same inclination and direction, to send a local alert, a remote alert or both.

7. System according to any of the previous claims wherein the mentioned monitoring device (1, 4) is programmed to alert when the individual is placed in an inclination and direction previously defined as inadequate.

8. System according to any of the previous claims wherein the mentioned monitoring device is programmed to, after detecting the individual's change of position and after detecting the individual's return to a previous position without having passed a predefined time for the individual to recover from the pressure of the previous position, send a local alarm, a remote alarm or both.

9. System according to any of the previous claims wherein the mentioned monitoring device (1, 4) or the mentioned acquisition unit (3, 6), or both, are programmed to gather and store relevant information from the temporal evolution of the individual's inclination and direction for posterior analysis.

10. System according to any of the previous claims for use in medicine.

11. System according to any of the previous claims,
for use in monitoring bedridden patients or in wheelchairs and respective prevention of pressure ulcers, wherein said system is for placing on the individual's torso (2) or waist (14); and/or
for use in monitoring patients suffering from dementia or motor limitation and respective accidents and fall prevention, wherein said system is for placing on the individual's thigh (5) or waist (14).

12. Method for monitoring and registering an individual's position, through a monitoring device (1, 4) coupled to the individual to be monitored, that comprises the following steps:
- obtaining the device's inclination through an inertial system, particularly, three or more accelerometers;
- obtaining the device's direction through a directional system, with three or more magnetometers,
- communicating the device's inclination and direction, and therefore the individual's inclination and direction, through a communication module.

13. Method for monitoring and registering an individual's position, according to claim 12, comprises determining indirectly the area of the individual's body that is under pressure and affected critical areas of the individual's body, assuming that an inclination of the back of a bed is changed whenever the mentioned device's inclination is kept for more than a predetermined period of time.

14. Method for monitoring and registering an individual's position, according to any of the claims 12 -13, comprises
for monitoring bedridden or in wheelchairs individual and the respective prevention of pressure ulcers, when after a maximum predefined time in the same position, and/or
for monitoring of individual suffering from dementia (5) or that have motor limitations and respective falls prevention, when after detecting the individual's change of position, and/or
for monitoring the gait detection and/or detection of individual's involuntary body movements, when the predefined limit of the predefined frequency range is reached,
and/or for monitoring the individual's spatial location, when a change of access points is detected,
comprises sending a local alert, remote alert or both.

15. Method for monitoring and registering an individual's position, according to any of the claims 12 - 14, comprises generating an alert when the individual is being placed in a position previously defined as inadequate.

16. Method for monitoring and registering an individual's position according to any of the claims 12 - 15, comprises gathering and storing of relevant information of the temporal evolution of the individual's position for posterior analysis.
